Europäisches Patentamt

European Patent Office

Office européen des brevets

(11) Veröffentlichungsnummer: **0 362 930**

**A1**

# (12) EUROPÄISCHE PATENTANMELDUNG

(21) Anmeldenummer: 89202423.3

(51) Int. Cl.⁵: **A61B 6/04**

(22) Anmeldetag: 27.09.89

(30) Priorität: 03.10.88 DE 3833594

(43) Veröffentlichungstag der Anmeldung:
**11.04.90 Patentblatt 90/15**

(84) Benannte Vertragsstaaten:
**DE FR GB NL**

(71) Anmelder: **Philips Patentverwaltung GmbH**
**Wendenstrasse 35 Postfach 10 51 49**
**D-2000 Hamburg 1(DE)**

(84) **DE**

Anmelder: **N.V. Philips' Gloeilampenfabrieken**
**Groenewoudseweg 1**
**NL-5621 BA Eindhoven(NL)**

(84) **FR GB NL**

(72) Erfinder: **Alich, Thomas**
**Hermelinweg 14a**
**D-2200 Elmshorn(DE)**
Erfinder: **Flisikowski, Peter**
**Joachim-Mähl-Strasse 1a**
**D-2000 Hamburg 61(DE)**
Erfinder: **Peemöller, Horst**
**Bengelsdorfstrasse 2**
**D-2000 Hamburg 71(DE)**

(74) Vertreter: **Kupfermann, Fritz-Joachim,**
**Dipl.-Ing. et al**
**Philips Patentverwaltung GmbH**
**Wendenstrasse 35 Postfach 10 51 49**
**D-2000 Hamburg 1(DE)**

(54) **Patientenliege für diagnostische Geräte.**

(57) Die Erfindung bezieht sich auf eine Patientenliege (1) für diagnostische Geräte, insbesondere für Tomographen, mit einem auflagefreien Auslegerarm (3), auf dem ein Patient in den diagnostischen Bereich des Gerätes schiebbar ist, wobei die Liege einen Grundkörper (9) aus faserverstärktem Epoxydharz und eine auf diesem vorgesehene Beschichtung (12) aufweist, wobei der Grundkörper (9) aus glasfaserverstärktem Kunststoff auf Epoxydharzbasis mit isotroper Glasfaserstruktur besteht, durch den sich in Längsrichtung Hohlräume (10) erstrecken, die mit einem Glasfaservlies ausgefüllt sind, das mit Epoxydharz getränkt ist, und wobei die Oberseite (11) mit einer faserverstärkten Kunststoff-Beschichtung (12) auf Epoxydharzbasis mit unidirektionalen Kunststoff-Fasern hoher Zugfestigkeit versehen ist.

Fig.4

## Patientenliege für diagnostische Geräte

Die Erfindung bezieht sich auf eine Patientenliege für diagnostische Geräte, insbesondere für Tomographen, mit einem auflagefreien Auslegerarm, auf dem ein Patient in den diagnostischen Bereich des Gerätes schiebbar ist, wobei die Liege einen Grundkörper aus faserverstärktem Epoxydharz und eine auf diesem vorgesehene Beschichtung aufweist.

Aus der US-PS 41 45 612 ist eine derartige Patientenliege bekannt, die aus Holz besteht und an ihrer Oberfläche mit einer faserverstärkten Polyesterharzschicht bedeckt ist. Die Patientenliege ist mit Rädern versehen und in den diagnostischen Bereich des Gerätes einfahrbar.

Die EP-Patentanmeldung 0 017 454 beschreibt eine Patientenliege, bei der der Grundkörper aus ausgehärtetem, Kohlefasern enthaltendem, thermisch härtbarem Harz besteht. Die Oberflächenbedeckung dieses Grundkörpers besteht aus einem epoxydharzgetränkten Kohlefasergewebe und ist damit elektrisch leitend. Diese elektrische Leitfähigkeit führt zu Feldverzerrungen des Hauptmagnetfeldes, also Feldlinien-Umlenkungen.

Die bekannten Patientenliegen weisen, soweit sie nicht unterstützt sind, eine relativ starke Durchbiegung auf, die von der Patientenlast abhängig ist. Soll die Patientenliege in Verbindung mit einem Tomographen, insbesondere mit einem 4-Tesla-Tomographen, eingesetzt werden, dan sind stärkere Durchbiegungen als beispielsweise 4 mm pro 2 m nicht zulässig.

Es ist Aufgabe der Erfindung, eine Patientenliege der eingangs erwähnten Art zu schaffen, bei der die Durchbiegung eines auflagefreien Auslegerarmes niedriger ausfällt als bei bekannten Liegen. Es sollen keine Wirbelströme auftreten und damit Ablenkungen der Feldlinien-Tomographen vermieden werden.

Die gestellte Aufgabe ist erfindungsgemäß dadurch gelöst, daß der Grundkörper aus glasfaserverstärktem Kunststoff auf Epoxydharzbasis mit isotroper Glasfaserstruktur besteht, durch den sich in Längsrichtung Hohlräume erstrecken, die mit Glasfaservlies angefüllt sind, das mit Epoxydharz getränkt ist, und daß die Oberseite mit einer faserverstärkten Kunststoffbeschichtung auf Epoxydharzbasis mit unidirektionalen Kunststoff-Fasern von hoher Zugfestigkeit versehen ist.

Eine derartige Patientenliege hat auf einer Länge von 2 m eine außerordentlich geringe Durchbiegung von maximal 4 mm. Die Formstabilität der Patientenliege ist unabhängig von der Umgebungstemperatur; sie läßt sich darüber hinaus universell einsetzen.

Nach einer weiteren Ausgestaltung der Erfindung ist vorgesehen, daß das glasfaserverstärkte Epoxydharzmaterial des Grundkörpers ein Glasfasergewebe aufweist, dessen E-Modul ca. 15.000 $N/mm^2$ beträgt. Ein solches Material eignet sich besonders als tragendes Material des Grundkörpers.

Nach einer weiteren Ausgestaltung der Erfindung ist vorgesehen, daß die unidirektionalen Fasern der Oberseitenbeschichtung eine hohe Zugfestigkeit und einen E-Modul von ca. 30.000 $N/mm^2$ aufweisen. Dieses Fasermaterial erhöht die Steifigkeit im Zugbereich der Liege.

Nach einer weiteren Ausgestaltung der Erfindung ist vorgesehen, daß das mit Epoxydharz getränkte Glasfaservlies, das im Handel unter der Bezeichnung "Coromat" erhältlich ist, geringe Dichte und eine hohe Druckfestigkeit von ca. 200 $N/mm^2$ im Verhältnis zur geringen Dichte von ca. 700 $kg/m^3$ aufweist. Dieses die Hohlräume ausfüllende Material verringert die Deformation der Grundkörperwandung unter Last bei einer relativ geringen Erhöhung des Gesamtgewichtes der Liege.

Schließlich ist nach einer weiteren Ausgestaltung der Erfindung vorgesehen, daß senkrechte Verstärkungsstreifen aus GFK das Widerstandsmoment in Belastungsrichtung erhöhen.

Die Erfindung wird anhand der Zeichnung näher erläutert. Es zeigen:

Fig. 1 eine Patientenliege nach der Neuerung in Seitenansicht,

Fig. 2 die Patientenliege nach Fig. 1 in Draufsicht,

Fig. 3 vier verschiedene Schnitte durch die Patientenliege, beginnend an einem rückwärtigen Verfahrteil und endend an dem vorderen Ende der Liege, wobei die Schnitte in Fig. 1 mit $III_1$, $III_2$, $III_3$ und $III_4$ bezeichnet sind,

Fig. 4 die Patientenliege in Verbindung mit einem 4-Tesla-Tomographen.

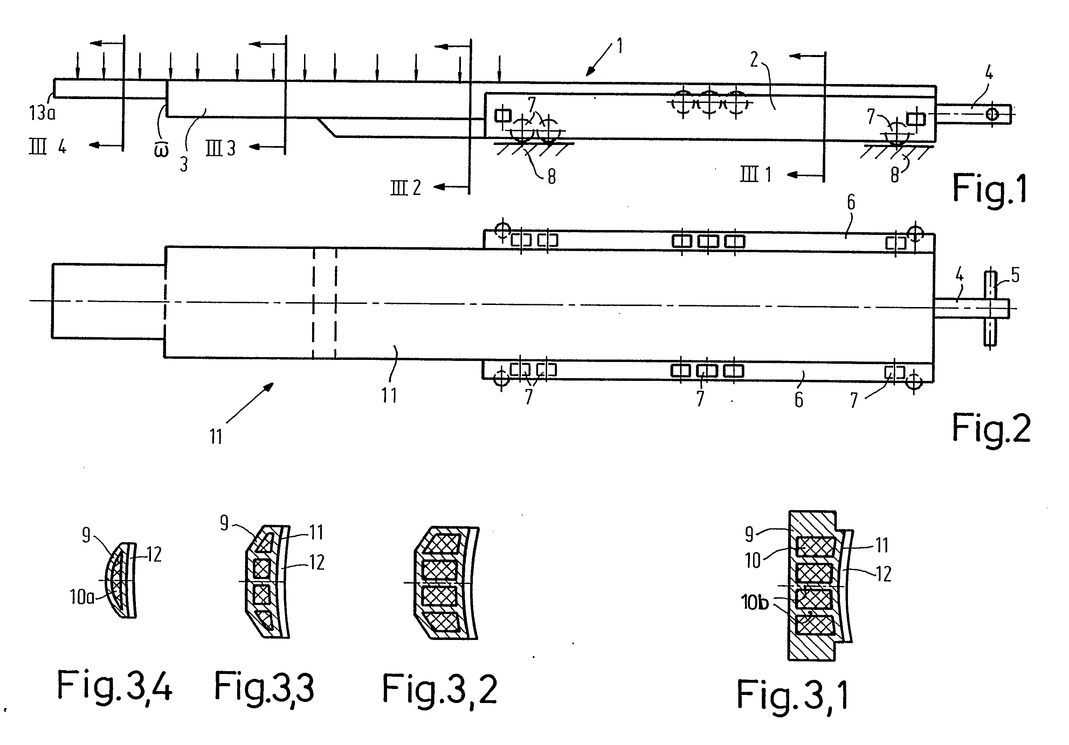

Die in Fig. 1 dargestellten Patientenliege 1 weist ein Verfahrteil 2 auf, das einen auflagefreien Auslegerarm 3 trägt. Die Patientenliege 1 ist von Hand verschieblich über einen Handgriff 4 mit einer Querstange 5.

Seitlich der Patientenliege 1 sind Verfahrvorrichtungen 6 vorgesehen, die Rollen 7 aufweisen, mit denen die Patientenliege 1 auf einer Auflage 8 verfahrbar ist. Die seitliche Anordnung der Verfahrvorrichtungen 6 ist deutlich aus Fig. 2 zu entnehmen.

Fig. 3 zeigt anhand der Schnitte III-III nach Fig. 1 den Querschnittsaufbau der Patientenliege anhand von vier einzelnen Schnitten im Bereich unterschiedlicher Querschnittsausbildungen. Man erkennt dabei anhand des Schnittes 3,1, daß die Patientenliege aus drei verschiedenen Materialien aufgebaut ist. Der Grundkörper 9 besteht aus einem glasfaserverstärkten Kunststoff auf Epoxydharzbasis mit einer isotropen Glasfaserstruktur (GFK). In Längsrichtung durch den Grundkörper 9 erstrecken sich Hohlräume 10, 10b, die mittels vertikaler Stege, die ebenfalls in Längsrichtung verlaufen, voneinander getrennt sind.

Diese Hohlräume 10 und 10a sind mit einem epoxydharzgetränkten Glasfaservlies ausgefüllt, das im Handel unter der Bezeichnung "Coromat" erhältlich ist. Dieses Material hat eine geringe Dichte in der Größenordnung von 700/ kg/m³ und eine hohe Druckfestigkeit in der Größenordnung von 200 N/mm². Aufgrund seiner geringen Dichte erhöht es das Gesamtgewicht der Patientenliege 1 nur wenig, verringert jedoch aufgrund seiner Druckfestigkeit die Deformation der Liegenwandung unter Last.

Auf die Oberseite 11 der Patientenliege ist eine Beschichtung 12 aufgebracht, die aus einem faserverstärkten Kunststoff auf Epoxydharzbasis besteht. Die Faserverstärkung innerhalb dieser Schicht besteht aus unidirektionalen Kunststoff-Fasern von hoher Zugfestigkeit (AFK). Diese Fasern sind im Handel beispielsweise unter dem Handelsnamen "Aramid" erhältlich. Diese speziellen Fasern verstärken und erhöhen die Zugfestigkeit der Patientenliege in dem oben gelegenen Zugbereich.

Man erkennt anhand der Schnitte in den Fig. 3,1 bis 3,4, daß die Hohlräume 10 getrennt sind mittels vertikaler, die Liege versteifender Stege 10b. Die Form der Hohlräume 10 der Stege 10b und die Form der Liege ändern sich mit kleiner werdendem Querschnitt, bleiben aber im Prinzip bis zum vorderen Ende 13, an das noch ein Kopfteil 13a angesetzt ist, gleich. Das Kopfteil 13a hat nur noch einen Hohlraum 10a und ist deutlich flacher ausgebildet.

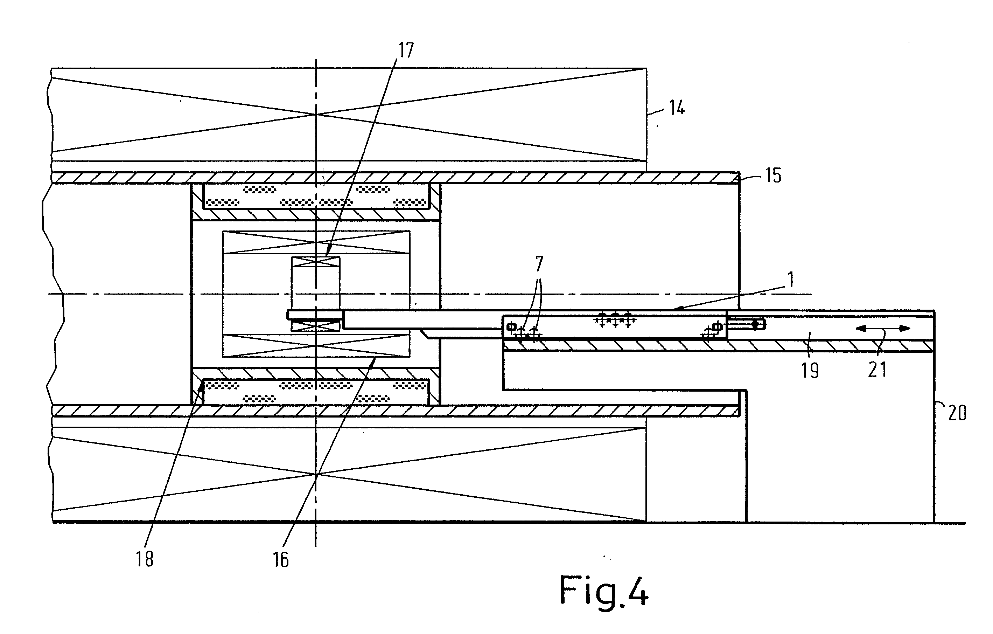

Fig. 4 zeigt, wie die Patientenliege in einen 4-Tesla-Magneten eines Computer-Tomographen einschiebbar ist. Durch den 4-Tesla-Magneten 14 erstreckt sich ein Tragrohr 15, in dem sich entweder eine HF-Ganzkörperspule 16 oder eine HF-Kopfspule 17 befindet. Zwischen dem Magneten 14 und den Gradientenspulen befindet sich ein Gradientenrohr 18. Die HF-Abschirmung ist nicht gezeichnet, ebenso wie das ganze Bild nur eine schematische Darstellung bildet. Die Patientenliege 1 wird auf einer Rollbahn 19, die auf einem Gestell 20 angeordnet ist, in Richtung eines Doppelpfeiles 21 verschoben. Entweder wird der Kopf des Patienten in die HF-Kopfspule 17 eingeschoben oder der ganze Patient auf dem Auflegearm in die HF-Ganzkörperspule.

Die Durchbiegung der Patientenliege, die wegen des langen 4-Tesla-Magneten sehr lang sein muß, liegt unterhalb von 4 mm und garantiert damit weitgehend verzerrungsfreie Schnittabbildungen.

## Ansprüche

1. Patientenliege (1) für diagnostische Geräte, insbesondere für Tomographen, mit einem auflagefreien Auslegerarm (3), auf dem ein Patient in den diagnostischen Bereich des Gerätes schiebbar ist, wobei die Liege einen Grundkörper (9) aus faserverstärktem Epoxydharz und eine auf diesem vorgesehene Beschichtung (12) aufweist, dadurch gekennzeichnet, daß der Grundkörper (9) aus glasfaserverstärktem Kunststoff auf Epoxydharzbasis mit isotroper Glasfaserstruktur besteht, durch den sich in Längsrichtung Hohlräume (10) erstrecken, die mit einem Glasfaservlies ausgefüllt sind, das mit Epoxydharz getränkt ist, und daß die Oberseite (11) mit einer faserverstärkten Kunststoff-Beschichtung (12) auf Epoxydharzbasis mit unidirektionalen Kunstoff-Fasern hoher Zugfestigkeit versehen ist.

2. Patientenliege nach Anspruch 1, dadurch gekennzeichnet, daß das glasfaserverstärkte Epoxydharzmaterial des Grundkörpers (9) ein Glasfasergewebe aufweist, dessen E-Modul ca. 15.000 N/mm² beträgt.

3. Patientenliege nach Anspruch 1, dadurch gekennzeichnet, daß die unidirektionalen Kunststoff-Fasern der Oberseitenbeschichtung (12) eine hohe Zugfestigkeit und einen E-Modul von ca. 30.000 N/mm² aufweisen.

4. Patientenliege nach Anspruch 1, dadurch gekennzeichnet, daß das mit Epoxydharz getränkte Glasfaservlies, das im Handel unter der Bezeichnung "Coromat" erhältlich ist, weist eine geringe Dichte von 700 kg/m³ und eine hohe Druckfestigkeit von 200 N/mm² auf.

5. Patientenliege nach Anspruch 1, dadurch gekennzeichnet, daß die Hohlräume (10) in Längsrichtung getrennt sind mittels vertikaler, versteifender Stege (10b).

6. Patientenliege nach Anspruch 1, dadurch gekennzeichnet, daß der den Auslegerarm (3) tragende Verfahrteil (2) der Liege (1) an seinen Längsseiten mit Rollvorrichtungen (6) versehen ist.

Fig.1

Fig.2

Fig.3,1

Fig.3,2

Fig.3,3

Fig.3,4

Fig.4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | US-A-4 145 612  (COOPER)<br>* Figuren 1,2; Spalte 2, Zeile 67 - Spalte 4, Zeile 55 *<br>--- | 1,3 | A 61 B    6/04 |
| A | US-A-4 262 204  (MIRABELLA)<br>* Figuren 3-6; Spalte 3, Zeile 66 - Spalte 4, Zeile 60 *<br>--- | 1,3 | |
| A | US-A-3 947 686  (COOPER et al.)<br>* Figuren 7,8; Spalte 3, Zeile 51 - Spalte 5, Zeile 39 *<br>----- | 1,3 | |

RECHERCHIERTE SACHGEBIETE (Int. Cl.5)

A 61 B
A 61 G

Der vorliegende Recherchenbericht wurde für alle Patentansprüche erstellt

| Recherchenort | Abschlußdatum der Recherche | Prüfer |
|---|---|---|
| DEN HAAG | 20-12-1989 | CHEN A.H. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)